# EUROPÄISCHE PATENTSCHRIFT

(11) **EP 0 047 877 B1**
(45) Veröffentlichungstag und Bekanntmachung des Hinweises auf die Patenterteilung: **23.01.1985**
(21) Anmeldenummer: 81106467.4
(22) Anmeldetag: 20.08.1981
(51) Int. Cl.: C07C 103/375, C07C 103/76, C07C 103/78, C07D 209/48, C07C 121/417, C07C 143/56, C07D 333/76, C07C 87/30, C07C 93/14, C07C 33/26, C07C 21/24

(54) **Biphenylverbindungen und Verfahren zu ihrer Herstellung**
Biphenyl compounds and process for their preparation
Composés du biphényle et procédé pour leur préparation

(30) Priorität: 02.09.1980 DE 3033002; 19.12.1980 DE 3048088
(43) Veröffentlichungstag der Anmeldung: 24.03.1982
(73) Patentinhaber: BAYER AG, 51368 Leverkusen (DE)
(72) Erfinder: Harnisch, Horst, Dr., D-5203 Much (DE)

## Beschreibung

Gegenstand der Erfindung sind Verbindungen der allgemeinen Formel 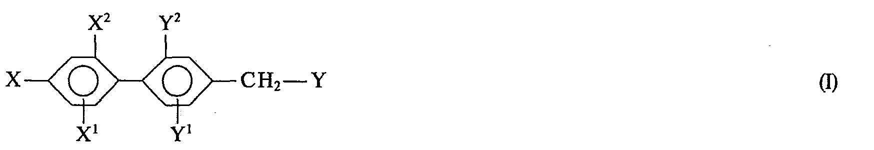 worin

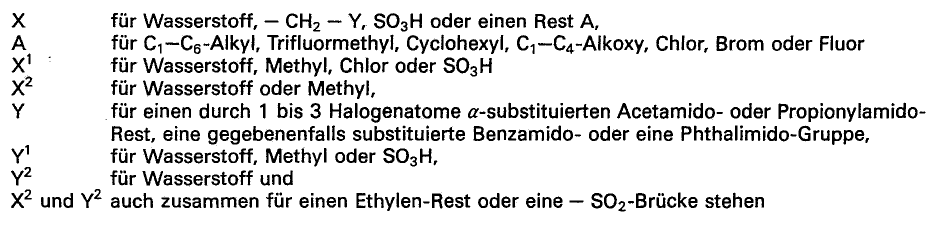

mit der Maßgabe, daß mindestens ein Rest Y für halogensubstituiertes Acetamido, Propionylamido oder für gegebenenfalls substituiertes Benzamido steht, wenn die Verbindungen frei von Sulfonsäuregruppen oder von - SO_{z}-Brücken sind.

Vorzugsweise beträgt die Gesamtzahl an Sulfonsäuregruppen maximal 2. Unter »Halogen« wird Chlor, Brom und Fluor, insbesondere Chlor, verstanden.

Geeignete Cₗ-C₆-Alkylreste A sind beispielsweise Methyl, Ethyl, Isopropyl, Tertiärbutyl, n-Butyl, Isoamyl und n-Hexyl. Bevorzugt sind C₁―C₄-Alkylreste.

Als C₁―C₄-Alkoxygruppen eignen sich vorzugsweise Methoxy, Ethoxy, Isopropoxy, n-Butoxy, Tertiärbutoxy, β-Methoxy-ethoxy und β-Ethoxy-ethoxy.

Als Beispiele für Y seien genannt: Chloracetamido, Dichloracetamido, Trichloracetamido, Trifluoracetamido, Bromacetamido, Benzamido und Phthalimido; bevorzugt sind Chloracetamido, Dichloracetamido und Trichloracetamido, insbesondere Chloracetamido, und Benzamido.

Benzamidoreste Y können bis zu 3 Kernsubstituenten enthalten, insbesondere C₁―C₄-Alkyl wie Methyl, Ethyl, Isopropyl, Tertiärbutyl; Halogen wie Chlor und Brom; C₁―C₂-Alkoxy wie Methoxy und Ethoxy; Nitro und Cyan. DasAmidstickstoffatom kann außerdem durch eine C₁―C₂-Alkylgruppe substituiert sein.

Geeignete substituierte Benzamidoreste Y sind beispielsweise 4-Methyl-, 2-Methyl-, 2,4-Dimethyl-, 2,4,6-Trimethyl-, 4-lsopropyl-, 4-Tertiärbutyl-, 4-Chlor-, 2-Chlor-, 2,4-Dichlor-, 3-Brom-, 4-Methoxy-, 4-Ethoxy-, 3-Nitro-, 4-Cyan-, N-Methyl- und N-Ethyl-benzamido. Die technisch wichtigsten sind außer dem unsubstituierten Benzamido das 4-Methyl- und 4-Chlorderivat.

Im Rahmen der Erfindung entspricht eine bevorzugte Gruppe von Verbindungen der Formel 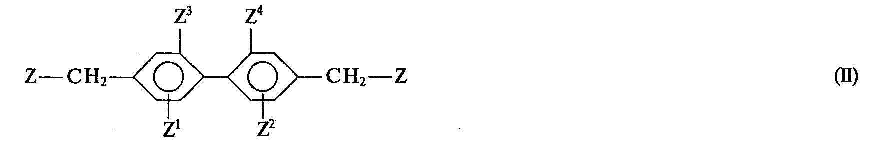 worin

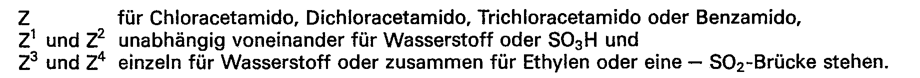

Eine weitere bevorzugte Gruppe von Verbindungen der Formel (I) entspricht der Formel 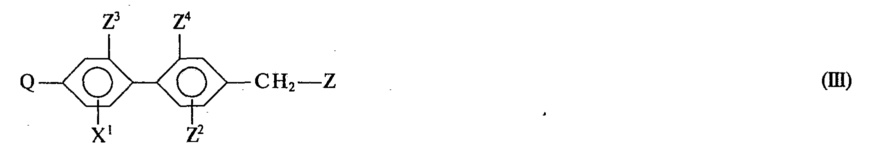 worin

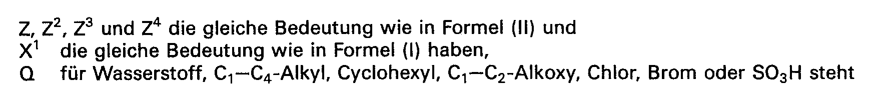

mit der Maßgabe, daß höchstens einer der Reste Q und X¹ SO₃H bedeutet.

Von besonderem technischen Wert sind diejenigen Verbindungen der Formel (11) und (111), in denen Z³ und Z⁴ für Wasserstoff und Z für Chloracetamido oder Benzamido stehen.

Ein vorteilhaftes Verfahren zur Herstellung von Verbindungen der Formel (I) ist dadurch gekennzeichnet, daß man Verbindungen der allgemeinen Formel 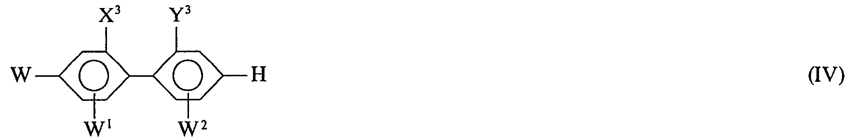 worin

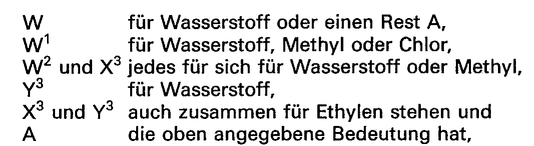

mit einem oder - im Falle W = H - gewünschtenfalls auch mit zwei Äquivalenten einer Verbindung der Formel 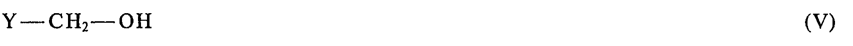 worin

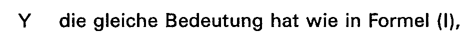

in Mischungen von Schwefelsäure und einer oder mehrerer niederer Fettsäuren, wie Essigsäure und Propionsäure, im Temperaturbereich von -10 bis +50°C, vorzugsweise bei 0-25°C, unter Abspaltung von Wasser kondensiert und gegebenenfalls anschließend mit einem sulfonierend wirkenden Mittel umsetzt.

Die Amidomethylierung von (IV) mit (V) entspricht dem Typ der Tscherniak-Einhorn-Reaktion [Houben-Weyl XI/1 (1957), 795; Org. Reactions 14 (1965), 52 und Synthesis 1970, 49].

Es ist als ausgesprochen überraschend zu bezeichnen, daß unter diesen Reaktionsbedingungen die Amidomethylierungsprodukte der Formel in hoher Ausbeute und guter Reinheit erhalten werden, da unter Standardbedingungen, d. h. beim Arbeiten in konzentrierter Schwefelsäure, diese Reaktion wenig selektiv verläuft und daher zu technisch unbrauchbaren Produkten führt. Ebenso überraschend ist es, daß andere gängige Methylolkomponenten als die der Formel wie beispielsweise Methylolformamid oder Methylolacetamid nicht so glatt reagieren.

Die Ausgangsverbindungen der Formel (IV) und (V) sind bekannt oder nach bekannten Methoden zugänglich.

Anstelle der Methylol-Verbindungen der Formel (V) können vorteilhaft auch Mischungen der entsprechenden Amide Y- H mit mindestens der äquivalenten Menge Formaldehyd, vorzugsweise Paraformaldehyd, eingesetzt werden. In einer weiteren Verfahrensvariante können anstelle von primären Amiden Y- H auch die entsprechenden Nitrile wie Chlor-, Dichlor-, Trichlor- oder Trifluor-acetonitril oder Benzonitril eingesetzt werden.

Nach der vorstehend dargelegten Verfahrensweise läßt sich auch das bisher nur mit 49 %der Theorie nach Ukr. Khim. Zh. 26, 277 (1960) erhältliche 4,4'-Bis-(phthalkimidomethyl)-biphenyl mit wesentlich höherer Ausbeute und Reinheit (93 %der Theorie) aus Biphenyl und zwei Äquivalenten Hydroxymethylphthalimid herstellen.

Sulfonsäurehaltige und/oder SO₂-Brücken enthaltende Vertreter der Formel (I) werden im Anschluß an die Amidomethylierung durch Umsetzung mit einem sulfonierend wirkenden Mittel wie Schwefelsäure (mit einem Gehalt von 0-65 % freiem S0₃) Chlorsulfonsäure, Pyridin-N-sulfonsäure oder S0₃ hergestellt, wobei das Sulfonierungsmittel in der Regel gleichzeitig als Reaktionsmedium dienen kann. Man arbeitet im Temperaturbereich von -10 bis 130°C, im allgemeinen vorzugsweise bei 0-30°C, bei ― SO₂-Brücken enthaltenden Verbindungen vorzugsweise bei 60-120°C.

In 100%iger Schwefelsäure reagieren SO₃H-gruppenfreie und SO₂-brückenfreie in 3-Stellung unsubstituierte Amidomethylverbindungen (I) in der Regel glatt zur entsprechenden Monosulfonsäuren, in 20 %igem Oleum dagegen überraschenderweise zum Sulfon (X² + Y² = ―SO₂ ―).

Die Verbindungen der Formel (I) sind wertvolle neue Zwischenprodukte für die Herstellung von technisch hochwertigen optischen Aufhellern, Farbstoffen und Kunststoffen.

Der erste Schritt, um aus den neuen Zwischenprodukten der Formel (I) zu optischen Aufhellern oder Kunststoffen zu gelangen, kann darin bestehen, diese zu den entsprechenden Aminomethylverbindungen der Formel 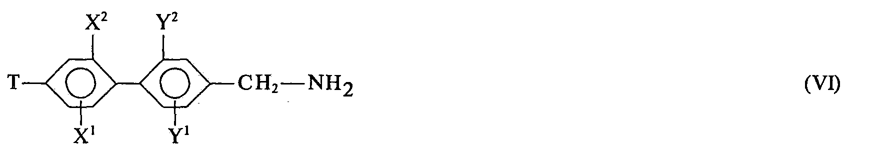 worin
T für Wasserstoff, - CH₂NH₂, S0₃H oder einen Rest A steht, wobei
A und die übrigen Reste die gleiche Bedeutung wie in Formel (I) haben

oder zu inneren oder sauren Salzen dieser Aminomethylverbindungen wie beispielsweise Hydrochloriden, Hydrobromiden, Hydrosulfaten oder Hydroperchloraten zu verseifen. Die Verseifung kann unter üblichen Bedingungen durch Erhitzen mit wäßrigen Mineralsäuren wie Salzsäure, Bromwasserstoffsäure, Schwefelsäure oder Phosphorsäure oder mit wäßrigem Alkalihydroxid wie Natronlauge oder Kalilauge auf Temperaturen von 30-200°C, vorzugsweise 80-160°C, vorteilhaft in Gegenwart eines mit Wasser mischbaren inerten organischen Lösungsmittels wie Ethylenglykol oder- bei saurer Verseifung - auch Eisessig oder Propionsäure erfolgen.

Phthalimidomethyl-Verbindungen können auch leicht mit Hydrazin in entsprechende Aminomethyl- verbindungen der Formel (VI) gespalten werden.

SO₂-Brücken enthaltende und sulfonsäuregruppenhaltige Verbindungen der Formel (VI) können auch aus den SO_{z}-brückenfreien bzw. den sulfonsäuregruppenfreien Verbindungen durch nachträgliche Umsetzung mit einem sulfonierend wirkenden Mittel hergestellt werden, wobei man als sulfonierend wirkende Mittel die oben beschriebenen verwendet und auch die dort angegebenen Reaktionsbedingungen anwendet. Bevorzugte Vertreter von SO₂-Brücken enthalten Verbindungen der Formel (VI) entsprechen der Formel 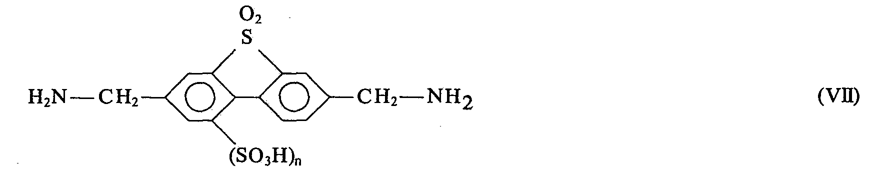 worin n für die Zahl 0 oder 1 steht.

Bevorzugte Vertreter von SO_{z}-brückenfreien, jedoch sulfonsäuregruppenhaltigen Verbindungen der Formel VI entsprechen der Formel 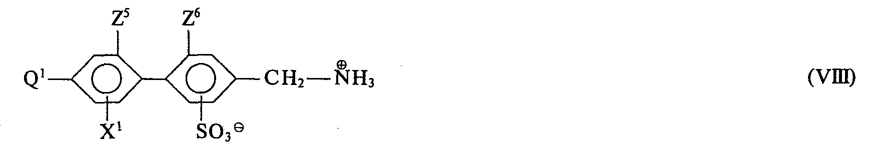 worin

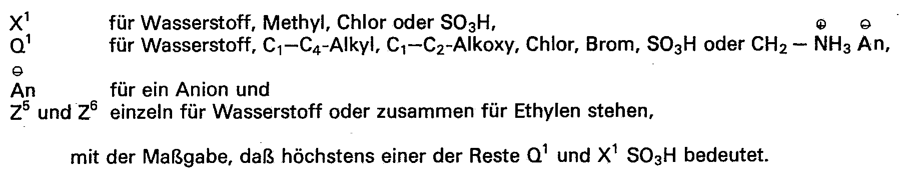

Unter Anionen An^{G} werden die Äquivalente gebräuchlicher Säureanionen wie Chloride, Sulfate, Hydrogensulfate, Nitrate, Phosphate, Hydrogenphoshate, Perchlorate, Chlorozinkate und Acetate verstanden. An^{⊖} kann aber auch durch eine im selben Molekül vorhandene SO₃⊖-Gruppe Q¹ oder X¹ vertreten sein.

Während sich für die Synthese von optischen Aufhellern alle Verbindungen der Formel (VI) vorteilhaft verwenden lassen, kommen für andere Einsatzgebiete speziellere Typen in Betracht.

Sulfogruppenfreie bifunktionelle Verbindungen der Formel (VI), in den T für- CH₂ - NH₂ steht, sind wertvolle Komponenten für Polyamide (US-PS 4 041 056) und für entzündungshemmende und fiebersenkende Mittel (US-PS 3 647 782). Sulfogruppenfreie monofunktionelle Verbindungen der Formel (VI) mit T= C₁―C₄-Alkoxy sind unmittelbare Vorstufen von Cholesterin- und Triglycerid-senkende Verbindungen (DE-OS 2 500 692).

Um zu optischen Aufhellern zu gelangen, werden die Aminomethyl-Verbindungen der Formel VI in einer weiteren Stufe beispielsweise mit salpetriger Säure oder mit einem Cₗ-C₅-Alkylester derselben und einer Mineralsäure unter N₂-Abspaltung in an sich bekannter Weise [Houben-Weyl XI/2 (1958), 133-137] zu den entsprechenden Hydroxymethyl-Verbindungen der Formel 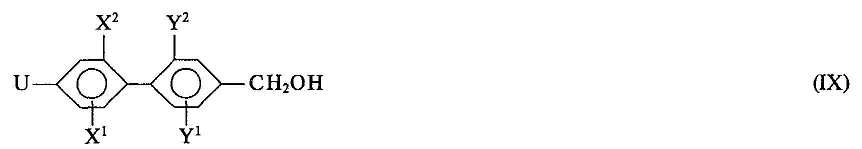 worin

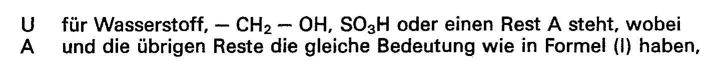

umgesetzt und anschließend entweder in an sich bekannter Weise [Houben-Weyl, VII/1 (1954) 177; Synthesis 1976, 88-89] schonend zu den Aldehyden der Formel 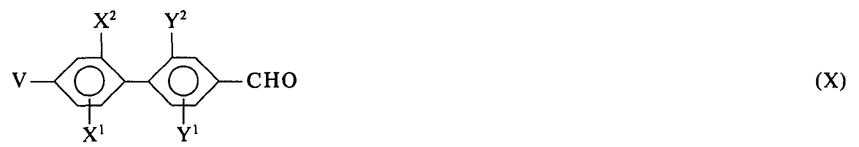 worin

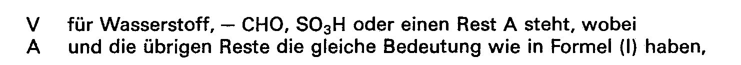

oxidiert oder mit Chlorwasserstoff oder anorganischen Säurechloriden wie Thionylchlorid, Phosphortrichlorid oder Phosphorpentachlorid in an sich bekannter Weise [Houben-Weyl, V/3 (1962) 830-837, 812-869, 899 und 905-911 zu den entsprechenden Chlormethylverbindungen der Formel 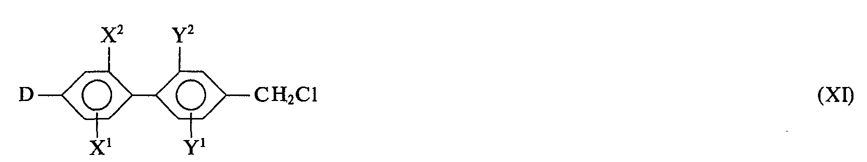 worin

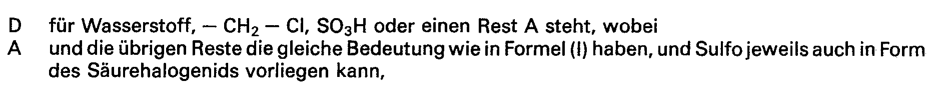

und diese dann mit Reagenzien wie Trialkylphosphit, Alkali-dialkoxyphosphonat oder Triphenylphosphin zu den entsprechenden phosphoraktivierten Methylverbindungen umgesetzt, die ebenso wie die Aldehyde der Formel (X) unmittelbare Vorstufen für konjugierte Ethylendoppelbindungen enthaltende optische Aufheller und Laserfarbstoffe mit Biphenyl-Struktur-Elementen sind (DE-AS 1 794 386, DE-OS 2 201 857, DE-OS 2 209 221, DE-OS 2 262 340, DE-OS 2 262 531, DE-OS 2 262 632, DE-OS 2 262 633, DE-OS 2 241 304, DE-OS 2 453 357, DE-OS 2 337 845, DE-OS 2 700 292, DE-OS 2 841 519, EP-OS 1 991 und US-PS 3 907 904).

Bischlormethyl-Verbindungen der Formel (XI) (D = CH₂Cl) liefern außerdem mit zwei Äquivalenten Salicylaldehyd wertvolle wichtige optische Aufheller der 4,4'-Bis-(benzofuran-2-yl)-biphenyl-Reihe (DE-OS 2 238 734).

Die als Ausgangsmaterialien benötigten Bischlormethylverbindungen sind bislang im technischen Maßstab nur durch Chlormethylierung des Biphenyls zugänglich gewesen, wobei jedoch gesundheitsschädigende Nebenprodukte entstehen, deren Handhabung bzw. Beseitigung einen erheblichen apparativen Aufwand erfordert.

Diese Nachteile weist das erfindungsgemäße Verfahren nicht auf.

Die Aldehyde der Formel (X) lassen sich aus den Aminomethylverbindungen der Formel (VI) auch auf anderen vorteilhaften Wegen herstellen, beispielsweise durch Sommelet-Oxidation mit Hexamethylentetramin im schwach sauren Medium (diese Methode ist beispielsweise in J. Chem. Soc. 1953, 1740-41 beschrieben), mit Wasserstoffperoxid in Gegenwart von Wolframat, eine in Chem. Ber. 93 (1960) 133 und Houben-Weyl X/4 (1 957) 123-124 beschriebene, zu den Aldoximen führende Methode, oder durch Kondensation mit einer Carbonylverbindung der Formel 0 = CR¹ R², worin

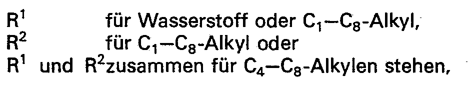

nachfolgende basisch katalysierte Umlagerung der - N = C-Doppelbindung in Konjugation zum Aromaten und saure Spaltung des erhaltenen Azomethins (diese Methode ist in der EP-OS 8 323 beschrieben).

Chlormethylverbindungen der Formel (XI) lassen sich auch vorteilhaft direkt aus den entsprechenden Aminomethylverbindungen der Formel (VI) durch Umsetzung mit Nitrosylchlorid nach der in Houben-Weyl V/3 (1962) 940 beschriebenen Methode herstellen.

Besonders rationell werden Chlormethylverbindungen der Formel (XI) in hoher Ausbeute dadurch hergestellt, daß man - unter Anwendung der v. Braun-Reaktion [Chem. Ber. 87 (1904) 2678, 2812, 3210 und 3583; Houben-Weyl V/3 (1962) 921 und JACS 84 (1962) 769] - Verbindungen der Formel I, in denen Y gegebenenfalls substituiertes Benzamido bedeutet, mit PCl₅ oder SOCl₂ umsetzt. Man arbeitet zweckmäßig in einem inerten organischen Lösungsmittel wie beispielsweise Chlorbenzol oder o-Dichlorbenzol im Temperaturbereich von 60-150°C, wobei Benzonitril abgespalten wird.

Den Chlormethylverbindungen der Formel (II) analoge Brommethylverbindungen können durch Umsetzung von (IX) mit PBr₃, POBr₃ oder PBr₅, durch Einwirkung von NO und Brom auf (VI) nach dem in Houben-Weyl V/4 (1960) 455 beschriebenen Verfahren oder durch Umsetzung von Benzamidomethylverbindungen (I) mit PBr₃ und Brom oder PBr₅ nach der in Houben-Weyl V/4 (1960) 451 beschriebenen v. Braun-Methode hergestellt werden.

Verbindungen der Formel (I), in denen Yfür den Chlor- oder Brom-acetamido-Rest stehen, lassen sich mit Alkalicyanid zu neuen Cyanacetamido-Verbindungen der Formel 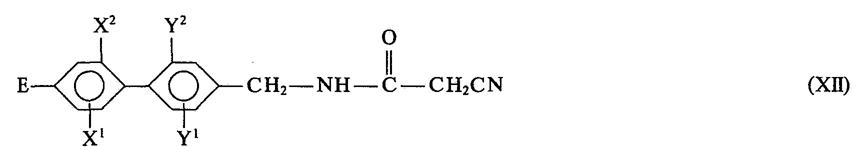 worin

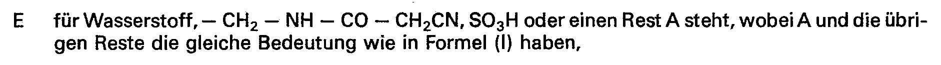

umsetzen. Diese sind neue kupplungs- und kondensationsfähige Farbstoff-Vorprodukte, unter denen die 4,4'-Bis-cyanacetamidomethyl-Derivate bevorzugt sind.

Die Umsetzung von I mit Alkalicyanid wird in einem polaren, mit Wasser mischbaren Medium wie Methanol, Ethanol, Isopropanol, Ethylenglykol, Ethylenglykolmonomethylether, vorzugsweise in einem dipolaraprotischen Lösungsmittel, wie Dimethylformamid oder Dimethylsulfoxid, im Temperaturbereich von 10-80°C, vorzugsweise 30-60°, durchgeführt.

Bemerkenswert ist übrigens, daß auch ein Teil der von den Formeln VI und X umfaßten Folgeprodukte bislang nicht in der Literatur beschrieben ist. Gleichwohl eignen sie sich in hohem Maße als Schlüsselprodukte für die oben angegebenen Einsatzgebiete. Es sind dies insbesondere solche Verbindungen der Formel VI und X, die eine Sulfogruppe und/oder eine - SO₂-Brücke (X² + Y²=―SO₂ ―) enthalten. Durch die Bereitstellung der erfindungsgemäßen Biphenyl-Verbindungen der Formel 1 bzw. des zu diesen Stoffen führenden Herstellungsverfahren werden somit rationelle Wege zu diesen wichtigen neuen Schlüsselprodukten eröffnet.

Von besonderer technischer Bedeutung sind unter diesen diejenigen, die der Formel 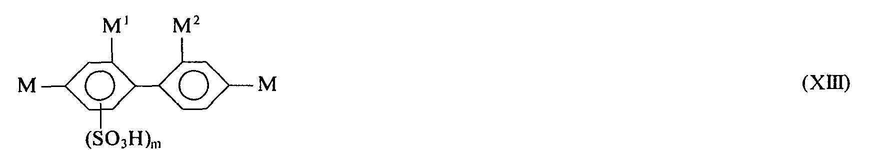 worin

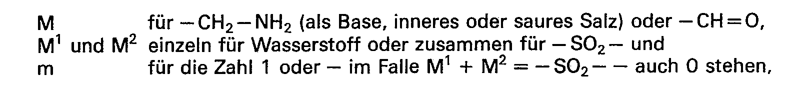

entsprechen.

Charakteristische Beispiele für derartige Folgeprodukte der Formel XIII sind:

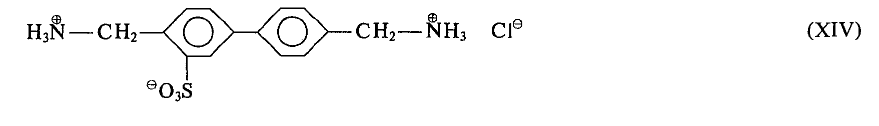

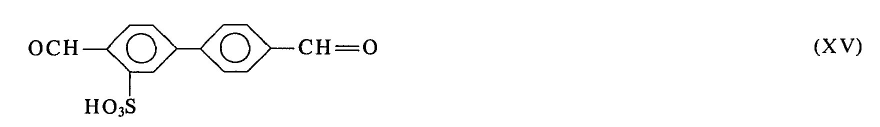

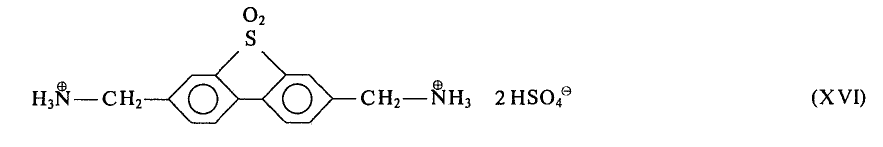

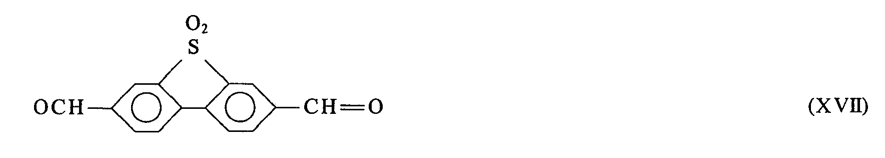

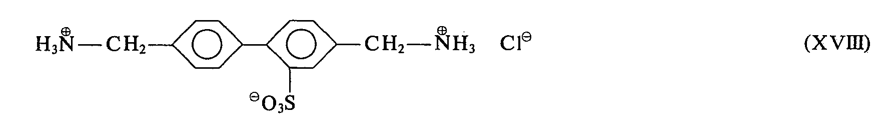

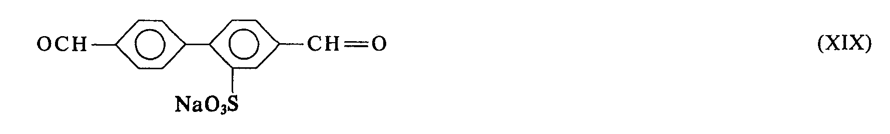

Wie bereits oben ausführlich dargelegt, erhält man diese Folgeprodukte aus den erfindungsgemäßen Biphenyl-Verbindungen der Formel I (nach der Umsetzung mit einem sulfonierend wirkenden Mittel) am einfachsten durch saure Verseifung zu den entsprechenden Aminomethyl-Verbindungen und gegebenenfalls anschließende Oxidation mit Urotropin.

### Beispiel 1

385 g Biphenyl werden in 1,5 I Propionsäure bei 30°C gelöst und bei 10-15°C unter starker Kühlung tropfenweise mit 0,9 I konzentrierter Schwefelsäure versetzt. Zur so erhaltenen Suspension gibt man bei 10-15°C 514 g Chloracetamid und 233 g Paraformaldehyd. Die Reaktionsmischung wird 11 Stunden bei 15°C verrührt und weitere 20 Stunden ohne zu rühren bei 15-18°C stehen gelassen. Anschließend setzt man 3 kg Eis hinzu und verrührt die Mischung 1 Stunde bei Raumtemperatur. Der kristalline Niederschlag wird über ein säurebeständiges Filter abgesaugt, mit 40 I Wasser neutral gewaschen und gut abgepreßt. Man erhält ca. 1 kg Verbindung der Formel 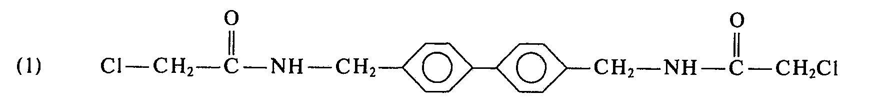 als feuchten Preßkuchen, der vorteilhaft in dieser Form weiterverarbeitet wird. Falls gewünscht, kann man die Substanz bei 50°C im Vakuum trocknen. Ausbeute: 820 g des farblosen Kristallpulvers. Eine aus Eisessig umkristallisierte Probe schmilzt bei 222-224°C, m/e = 364 (M^{⊕}, Dichlor).

In analoger Weise werden die in der folgenden Tabelle aufgeführten Verbindungen der allgemeinen Formel 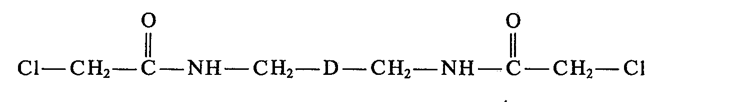 hergestellt:

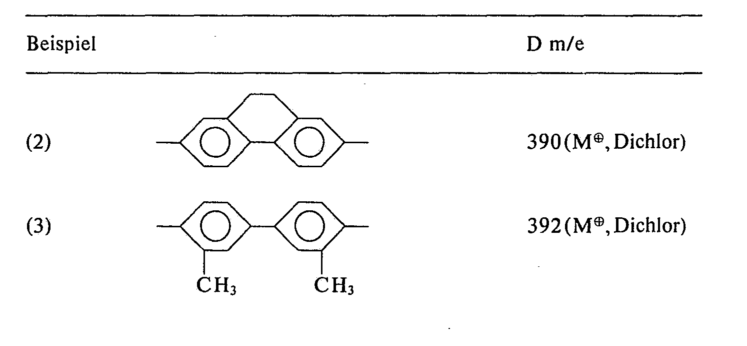

### Beispiel 4

38,5 g Biphenyl werden in 150 ml Propionsäure bei 35°C gelöst und bei 10-15°C unter starker Kühlung tropfenweise mit 90 ml konzentrierter Schwefelsäure versetzt. Zur so erhaltenen Suspension gibt man bei 10-1 5°C 70,5 g Dichloracetamid und 23,3 g Paraformaldehyd. Die Reaktionsmischung wird 11 Stunden bei 15°C verrührt und weitere 20 Stunden, ohne zu rühren, bei 15-18C stehen gelassen. Anschließend setzt man 300 g Eis hinzu und verrührt die Mischung 1 Stunde bei Raumtemperatur. Der kristalline Niederschlag wird über ein säurebeständiges Filter abgesaugt, mit 0,5 I Wasser neutral gewaschen und gut abgepreßt. Nach dem Trocknen bei 50°C im Vakuum werden 97 g Verbindung der Formel 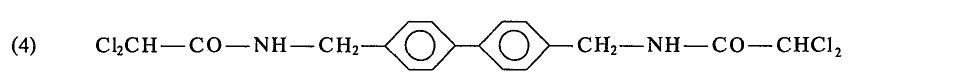 als farbloses Kristallpulver erhalten. Schmelzpunkt: 227-228°C [aus Eisessig; m/e = 432 (M^{D}, Tetrachlor)].

### Beispiel 5

Ersetzt man in Beispiel 4 Dichloracetamid durch eine äquivalente Menge Trichloracetamid, so erhält man 114 g Verbindung der Formel 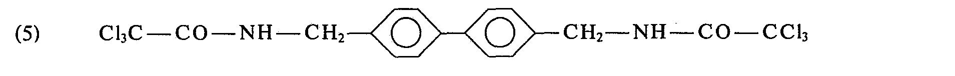 m/e = 500 (M^{⊕}, Hexachlor).

Ersetzt man Dichloracetamid durch a-Chlor-propionsäureamid oder Bromacetamid, so erhält man in analoger Weise 4,4'-(a-Chlorpropionylamidomethyl)-biphenyl, m/e = 392 (M^{⊕}. Dichlor) bzw. 4,4'-(Bromacetamidomethyl)-biphenyl, m/e = 454 (M^{⊕}).

### Beispiel 6

308 g Biphenyl werden in 1750 ml Eisessig unter Erwärmen gelöst und unter starker Kühlung bei 20-25°C tropfenweise mit 1500 ml konzentrierter Schwefelsäure versetzt. Anschließend fügt man 533 g Benzamid und 132 g Paraformaldehyd hinzu, verrührt die Mischung 20 Stunden bei Raumtemperatur und trägt sie auf 10 I Eiswasser aus. Nach 2-stündigem Verrühren wird der kristalline Niederschlag abgesaugt, mit Wasser neutral gewaschen und bei 50°C im Vakuum getrocknet. Man erhält 840 g Verbindung der Formel 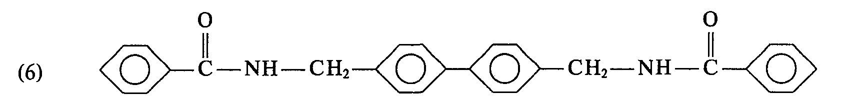 Schmelzpunkt: 247°C (aus Eisessig); m/e = 420 (M^{G})

In analoger Weise werden die folgenden Verbindungen der allgemeinen Formel

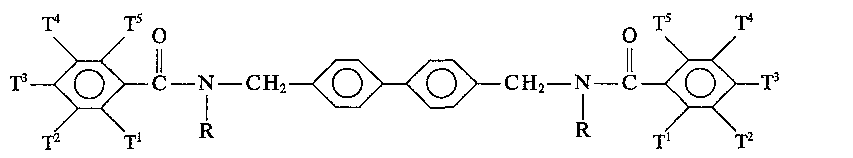

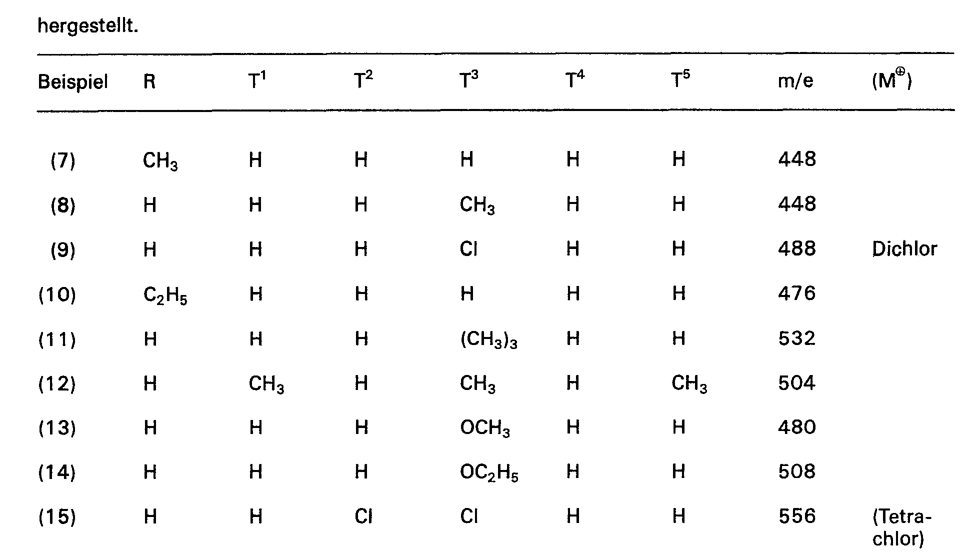

### Beispiel 16

308 g Biphenyl werden in 1750 ml Eisessig unter Erwärmen gelöst und unter starker Kühlung bei 20-25°C tropfenweise mit 1300 ml konzentrierter Schwefelsäure versetzt. Anschließend fügt man 789 g N-Methylolphthalimid hinzu, verrührt die Mischung 20 Stunden bei Raumtemperatur und tropft unter starker Kühlung ebenfalls bei Raumtemperatur 2,5 I Wasser zu. Nach 1-stündigem Verrühren wird der kristalline Niederschlag abgesaugt, mit Wasser neutral gewaschen und bei 80°C im Vakuum getrocknet. Man erhält 878 g (93 % der Theorie) Verbindung der Formel 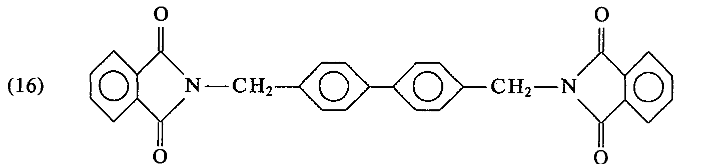 Schmelzpunkt: 299°C (aus Eisessig); m/e = 472 (M^{⊕}).

### Beispiel 17

38,5 g Biphenyl werden in 220 ml Propionsäure gelöst und auf 10°C gekühlt. Unter starker Kühlung tropft man bei 10-15°C 30 ml konzentrierte Schwefelsäure hinzu und versetzt die Lösung anschließend mit 37,4 g Chloracetamid und 12 g Paraformaldehyd. Nach 20stündigem Rühren bei 1 5°C trägt man die Reaktionsmischung auf 1 I Eiswasser aus, saugt den kristallinen Niederschlag ab und wäscht ihn mit Wasser neutral. Der feuchte Preßkuchen wird zur Entfernung des Wassers mit 250 ml Toluol am Wasserabscheider gekocht, wobei eine klare Lösung entsteht. Nach dem Abkühlen der Lösung wird der kristalline Niederschlag abgesaugt, mit Petroläther gewaschen und bei 40°C im Vakuum getrocknet. Man erhält 8 g Verbindung der Formel 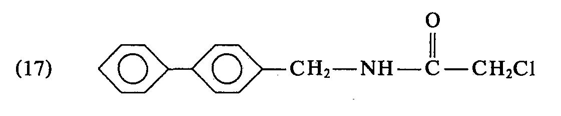 Schmelzpunkt: 149-150°C; mle = 259 (M , Monochlor).

Aus der Toluol Mutterlauge werden nach dem Abdampfen des Lösungsmittels 62 %des eingesetzten Biphenyls wiedergewonnen.

### Beispiel 18

115 g 4-Methoxy-biphenyl werden in 2,5 I Propionsäure mit 64,5 g Chloracetamid und 29,5 g Paraformaldehyd versetzt und auf 85°C erwärmt, wobei eine klare Lösung entsteht. Diese wird auf 20°C abgekühlt, unter starker Kühlung bei 20-25°C tropfenweise mit 135 ml konzentrierter Schwefelsäure versetzt, 5 Stunden bei 20-25°C verrührt und dann auf 5 I Eiswasser ausgetragen. Der kristalline Niederschlag wird abgesaugt, mit Wasser neutral gewaschen und bei 50°C im Vakuum getrocknet. Man erhält 164 g Verbindung der Formel 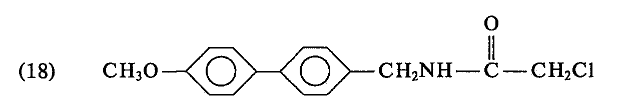 Schmelzpunkt: 132-133°C (aus Di-sek.-butylether): m/e = 289 (M^{⊕}, Monochlor).

In analoger Weise werden die in der folgenden Tabelle aufgeführten Verbindungen der allgemeinen Formel 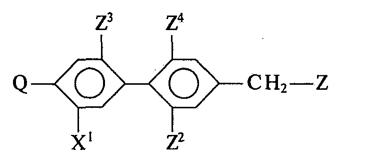 hergestellt:

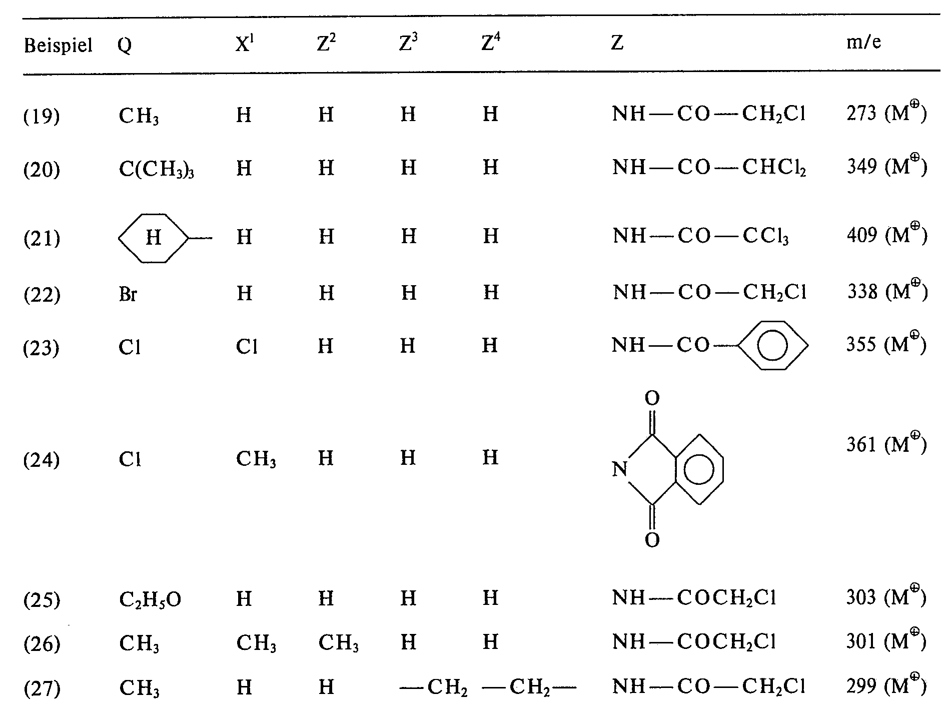

### Beispiel 28

1/10 des nach Beispiel 1 hergestellten feuchten Preßkuchens an Verbindung (1) werden in 680 ml Dimethylformamid suspendiert, mit 85 ml 30 Vol.-% Natriumcyanidlösung 4 Stunden auf 40-45°C erwärmt, auf 4,5 Eiswasser ausgetragen und verrührt. Der kristalline Niederschlag wird abgesaugt, mit Wasser gewaschen und bei 80°C im Vakuum getrocknet.
Ausbeute: 78,5 g Verbindung der Formel

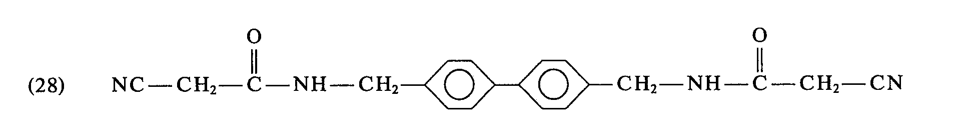

Schmelzpunkt: 270-271°C (aus Eisessig); m/e = 346 (M^{⊕}).

### Beispiel 29

60 g Verbindung der Formel (1) werden unter Rühren und Kühlung bei 5-20°C nach und nach in 600 g 100%idge Schwefelsäure eingetragen, 1 Tag bei 20-25°C verrührt und auf 1 kg Eis ausgetragen. Nach 1 stündigem Rühren wird der kristalline Niederschlag abgesaugt, mit Eiswasser gewaschen und bei 60°C im Vakuum getrocknet. Man erhält 81,2 g Verbindung der Formel

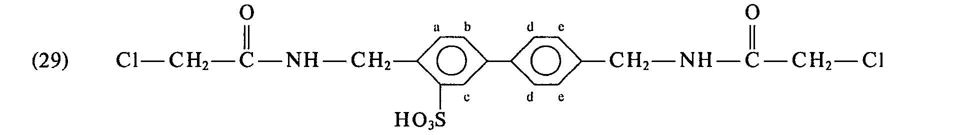

Sie kann durch Verrühren mit Aceton gereinigt werden.
¹H-NMR ([D6]-DMSO/TMS):
   δ = 4,12 (S; 4 H, CH₂Cl), 4,30 (brd, J = 6 Hz; 4H, ―CH2―NH―), 7,17 (d, J = 8 Hz; 1H, Hₐ),
   AA'BB'-Signal (δ_{A}= 7,50, δ_{B} = 7,17, J₁ = 8 Hz, J₂ nicht ausgelöst; 4H, Hₐ + H_{d}), 7,50 (dd, J₁ = 8 Hz, J₂ nicht ausgelöst; 1 H, H_{b}); 7,85 (d, J = 1,5 Hz; 1 H, Hₑ), 8,76 (t, J = 6 Hz; 2 H, NH).

### Beispiel 30

500 g Verbindung der Formel (1), hergestellt nach Beispiel 1, anschließendes Ausrühren mit Eisessig und Trocknen bei 80°C im Vakuuum, werden bei 10-20°C portionsweise in 2,6 kg Oleum (20%freies S0₃) eingetragen, 18 Stunden bei Raumtemperatur verrührt und auf Eis ausgetragen. Der kristalline Niederschlag wird abgesaugt, mit Wasser gewaschen und gewünschtenfalls bei 60°C im Vakuum getrocknet. Man erhält 580 g Verbindung der Formel 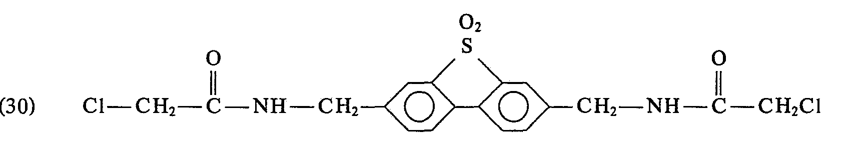 Schmelzpunkt: 287°C unter Zersetzgl (aus Dimethylformamid).
¹H-NMR ([D₆]-DMSO/TMS):
   δ = 4,23 (S; 4 H, 2 CH₂Cl), 4,50 (d, J = 6 Hz; 4 H, 2 CH₂NH), 7,71 (dd, J₁ = 8 Hz; J₂ Hz; 2 H) 7,88 (d, J = 2 Hz; 2 H), 8,17 (d, J = 8 Hz; 2 H) 8,92 (t, J = 6 Hz; 2 H, 2 CH₂― NH―).

Setzt man anstelle der Verbindung (1) gleiche Gewichtsteile der Verbindung (6) ein, so erhält man in analoger Weise 520 g Verbindung der Formel

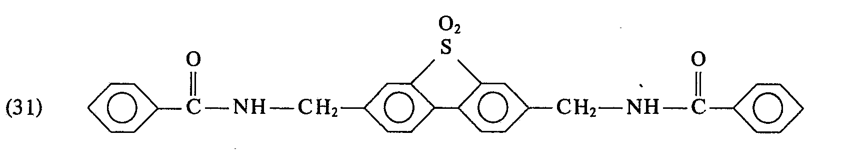

Die Reinigung erfolgt durch Auskochen mit Ethylenglykolmonomethylether:
C₂₈H₂₂N₂SO₄ (482,57):

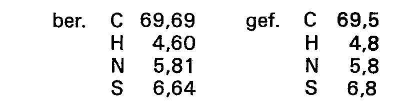

### Beispiel 32

50 g Verbindung der Formel (30) werden in 250 g Schwefelsäure (100 %ig) unter Rühren gelöst, tropfenweise mit 250 g Oleum (65 %ig freies SO₃) versetzt, 4 Std. auf 100°C erhitzt, auf 1,5 kg Eis ausgetragen und mit konzentrierter Natronlauge neutralisiert. Der kristalline Niederschlag wird über ein säurebeständiges Filter abgesaugt, mit 5 %ig Kochsalzlösung gewaschen und bei 50°C im Vakuum getrocknet. Man erhält 48 g Verbindung der Formel 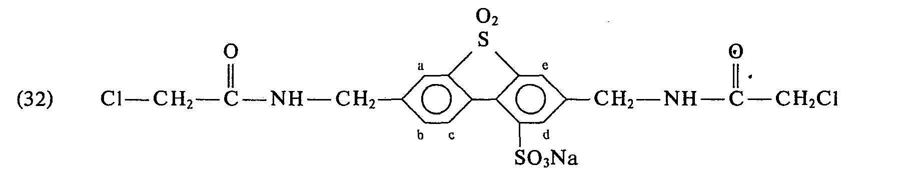 als farbloses, wasserlösliches Kristallpulver.
¹H-NMR ([D₆]-DMSO/TMS):
   δ = 4,20 (s; 4 H, 2 CH₂Cl), 4,45 (d, J = 6 Hz; 4 H, 2―CH₂ NH―), 7,66 (dd, J₁ = 9 Hz; J₂ = 2 Hz; 1 H, H_{b}), 7,79 (d, J = 2 H; 1 H, Hₐ), AB-Signal (δ_{A} = 8,19, δ_{B} = 7,79, J = 2 Hz; 2 H, H_{d} + Hₑ), 8,92 (t, J = 6 Hz; 2 H, ―CH₂― NH―), 9,26 (d, J = 9 Hz; 1 H, H_{c}).
¹H-NMR (D₂O/TMS):
   δ = 7,70 (dd, J₁ = 8 Hz; J₂ = 1,5 Hz; 1 H, H_{b}), 7,84 (d, J = 1,5 Hz; 1 H, Hₐ), AB-Signal (δ_{A} = 8,23, δ_{B} = 7,97, J = 1,5 Hz; 2H. H_{d} + Hₑ), 8,87 (d, J = 8 Hz; 1 H, H_{c}).

### Beispiel 33

In eine Mischung aus 1,6 I Eisessig und 1,6 I konzentrierte Salzsäure wird die Gesamtmenge des nach Beispiel 1 hergestellten feuchten Preßkuchens an Verbindung (1) eingetragen und 18 Stunden auf 85°C erwärmt. Nach dem Abkühlen auf Raumtemperatur wird die Mischung 2 Stunden bei 20°C verrührt. Der kristalline Niederschlag wird über ein säurebeständiges Filter abgesaugt und mit 2 I Ethanol zügig gewaschen. Nach dem Trocknen im Vakuum bei 80°C werden 506 g Verbindung der Formel 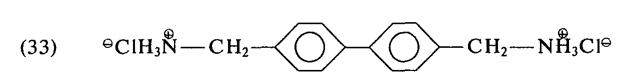 erhalten.
¹H-NMR (D₂O/TMS):
   δ= 4,30 (s; 4 H, 4,82 (s; HDD in - N H₃), AA'BB'-Signal (δ_{A}= 7,85, δ_{B} = 7,60, J, = 8,5 Hz, J₂ nicht aufgelöst; 8 arom.H).
m/e (Base) = 212 (M^{⊕})

In analoger Weise werden die Verbindungen der Formel (2) bis (5) verseift.

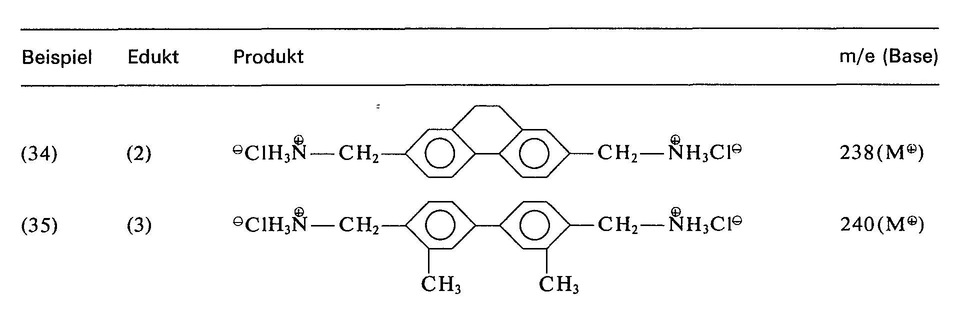

### Beispiel 36

100 g Verbindung der Formel (29) werden in einer Mischung aus 400 ml konz. Salzsäure und 400 ml Eisessig 2 Stunden unter Rückfluß zum Sieden erhitzt. Das Lösungsmittel wird im Wasserstrahlvakuum abgedampft. Der Rückstand wird nach dem Abkühlen auf 20°C mit 500 ml Aceton 2 Stunden verrührt. Der kristalline Niederschlag wird abgesaugt, mit Aceton gewaschen und 30°C im Vakuum getrocknet. Man erhält 72 g Verbindung der Formel 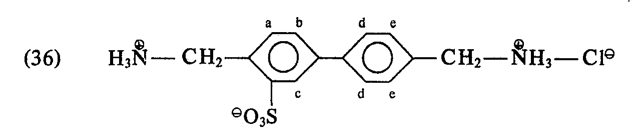 als farbloses, gut wasserlösliches Kristallpulver.
¹H-NMR ([D₆]-DMSO/TMS):
   δ= 4,25 (s; 2 H, CH₂), 4,30 (s; 2 H, CH₂), 7,33 (d, J = 8 Hz; 1 H, Hₐ), 7,52 (s; 4 H, s H_{d} + 2 Hₑ), 7,68 (dd, J₁ = 8 Hz, J₂ = 2 Hz; 1 H, H_{b}), 8,16 (d, J = 2 Hz; 1 H, H_{c}).

### Beispiel 37

500 g Verbindung der Formel (30) werden in einer Mischung aus 2,5 I konzentrierter Salzsäure und 2,5 I Eisessig 4 Stunden unter Rückfluß zum Sieden erwärmt und dann abgekühlt. Der kristalline Niederschlag wird abgesaugt, mit Ethanol gewaschen und bei 50°C im Vakuum getrocknet. Man erhält 335 g Verbindung der Formel 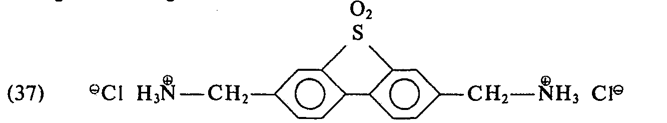 als farbloses, leicht wasserlösliches Kristallpulver.
¹H-NMR ([D₆]-DMSO/TMS):
   δ = 4,23 (s; 4 H, 2 CH₂), 8,0 (dd, J₁ = 8 Hz; J₂ = 2 Hz; 2 H, 8,21 (d, J = nicht aufgelöst;
   2 H), 8,28 (d, J = 8 Hz; 2 H), 8,85 (s; 6 H, 2 N H₃).

In analoger Weise wird die Verbindung der Formel (32) zur entsprechenden Bisaminomethylverbindung der Formel 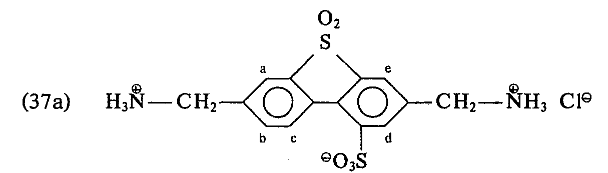 verseift.
¹H-NMR ([D₆]-DMSO/TMS):
   δ = 4,16 (s; 2 H, CH₂). 4,25 (s; 2 H, CH₂), 7,92 (dd, J₁ = 8 Hz, J₂ = 1,5 Hz; 1 H, H_{b}), 8,12 (d, J = 1,5 Hz; 1 H, Hₐ), AB-Signal (δ_{A} = 8,37, δ_{B} = 8,16, J = 2 Hz; 2 H, H_{d} + Hₑ), 8,68 (br. s; 2N H₃), 9,35 (d, J = 8 Hz; 1 H, H_{c}).

### Beispiel 38

Die Gesamtmenge der nach den Angaben des Beispieles 18 erhaltenen Verbindung der Formel (18) als feuchter Preßkuchen wird in einer Mischung aus 825 ml Eisessig und 275 ml konzentrierter Salzsäure 18 Stunden auf 85°C erwärmt. Anschließend dampft man das Lösungsmittel im Vakuum ab und verrührt den Rückstand mit 500 ml Aceton.

Der kristalline Niederschlag wird abgesaugt, mit Aceton gewaschen und bei 50°C im Vakuum getrocknet. Man erhält 105 g Verbindung der Formel 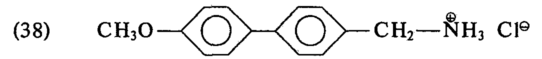 m/e (als Base) = 213.

In analoger Weise werden die Verbindungen (19), (20), (21), (22), (25), (26) und (27) zu den entsprechenden sauren Aminomethyl-Verbindungen verseift.

### Beispiel 39

142,5 g eines nach Beispiel 33 hergestellten Präparates der Formel (33) werden bei 5-15°C in 690 g 100%ige Schwefelsäure eingetragen, wobei Chlorwasserstoff entweicht. Man verrührt die Lösung 1 Stunde, tropft dann bei 10-20°C unter Kühlung 690 g Oleum (mit 20% freiem S0₃) hinzu, läßt die Lösung 3 Tage bei Raumtemperatur stehen und trägt sie auf 3 kg Eis aus. Der kristalline Niederschlag wird über ein säurebeständiges Filter abgesaugt, mit Eiswasser gewaschen und bei 80°C im Vakuum getrocknet.

Man erhält 116 g Verbindung der Formel 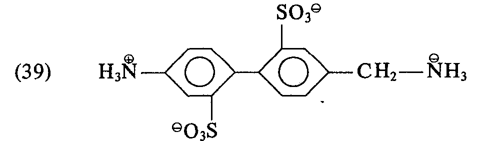 Schmelzpunkt: 327°C (unter Zersetzung)
¹H-NMR ([D₆]-DMSO):
   ö = 4,28 (s; 4 H, CH₂), 8,06 (dd, J₁ = 8,5 Hz, J₂ = 1 Hz; 2 H), 8,23 (d, J = 1 Hz; 2 H), 8,43 (d, J = 8,5 Hz; 2 H) 8,45 (br. s; 6 H, - N H₃).

Zur Disulfonierung von Beispiel (34) verfährt man in analoger Weise, arbeitet aber bereits nach 20stündigem (statt 3tägigem) Stehen bei Raumtemperatur auf. Man erhält auf diese Weise die Verbindung der Formel 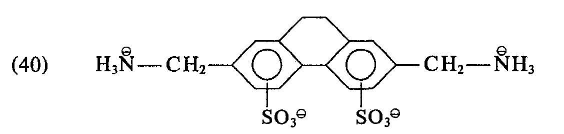 als farbloses, >300°C unter Zersetzung schmelzendes Kristallpulver.

### Beispiel 41

170 g Verbindung der Formel (33) werden in 3,6 Wasser bei 85°C gelöst und unter Rühren nach und nach mit insgesamt 216 g Natriumnitrit versetzt. Anschließend tropft man - immer bei 85°C - innerhalb von 1 Stunde 240 g konzentrierte Salzsäure unter die Oberfläche der Lösung. Nach dem Abkühlen wird der kristalline Niederschlag abgesaugt, mit Wasser gewaschen und zur Entfernung des Wassers mit 1 I Essigsäurebutylester am Wasserabscheider gekocht. Nach dem Abfiltrieren ungelöster Anteile wird die Lösung stark abgekühlt. Der kristalline Niederschlag wird abgesaugt, mit Petrolether gewaschen und bei 50°C im Vakuum getrocknet. Man erhält 100,5 g Verbindung der Formel 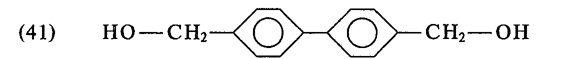 Schmelzpunkt: 188°C; m/e = 214 (M^{⊕}).

### Beispiel 42

53,5 g Verbindung der Formel (41) werden in 200 ml Toluol suspendiert, unter Rühren tropfenweise mit 75 g Thionylchlorid versetzt und 2 Std auf 40°C erwärmt. Anschließend wird das Lösungsmittel zusammen mit dem überschüssigen Thionylchlorid im Vakuum abdestillliert. Man erhält als Rückstand 63 g Verbindung der Formel 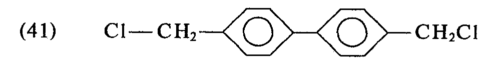 m/e = 250 (M^{⊕}, Dichlor). Sie wird ohne Zwischenisolierung weiterverarbeitet:
A) Nach den Angaben der DE-AS 1 794386, Spalte 18 wird 4,4'-Bis-(chlormethyl)-biphenyl (42) durch Umsetzung mit Trimethylphosphit zu 4,4'-Bis-(dimethoxy-phosphonomethyl)-biphenyl umgesetzt und dieses mit Benzaldehyd-2-sulfonsäure zu 4,4'-Bis-(2-sulfostyryl)-biphenyl, einem in Waschflotten applizierten optischen Aufheller für Baumwolle und Polyamid, kondensiert.
B) Nach den Angaben der DE-OS 2 238 734 wird 4,4'-Bis-(chlormethyl)-biphenyl (42) mit Salicylaldehyd zu 4,4'-Bis-(benzo[b]furan-2-yl)-biphenyl und dieses weiter mit Oleum zu einem Gemisch der entsprechenden Tri- und Tetrasu lfonsäure umgesetzt. Auch dieses dient zum optischen Aufhellen von Baumwolle und Polyamid.

### Beispiel 43

150 g Verbindung der Formel (6) werden in 900 ml o-Dichlorbenzol suspendiert, mit 164,3 g Phosphorpentachlorid versetzt und 1,5 Std. auf 110-115°C erhitzt, wobei man einen Teil des entstehenden POCI₃ abdestillieren läßt. Anschließend destilliert man im Wasserstrahlvakuum das restliche POCI₃ zusammen mit dem Lösungsmittel und dem entstandenen Benzonitril ab (Badtemperatur bis 130°C). Der Rückstand (98 g) wird aus 450 ml Methylcyclohexan umkristallisiert. Man erhält 78 g Verbindung der Formel (42).

Ein ähnliches Ergebnis wird erhalten, wenn man anstelle von PCl₅ 115 ml Thionylchlorid einsetzt, 1,5 Stunden auf 80°C erhitzt, den SOCl₂-Überschuß abdestilliert, die Mischung noch 10 Stunden auf 150°C erhitzt, das Lösungsmittel und Benzonitril im Vakuum abdestilliert und den Rückstand wie oben beschrieben umkristallisiert.

Ersetzt man Verbindung (6) durch eine der Verbindungen der Formel (7) bis (15), so erhält man in analoger Weise jeweils 4,4'-(Bischlormethyl)-biphenyl (42) in ähnlich hoher Ausbeute.

### Beispiel 44

50,2 g Verbindung der Formel (42) werden in 400 ml Dimethylformamid bei 40°C mit 70 ml 30%ig NaCN-Lösung versetzt und 2 Stunden bei 40-45°C verrührt. Nach dem Austragen auf 2 I Eiswasser wird der kristalline Niedersch lag abgesaugt, mit Wasser gewaschen und bei 50°C im Vakuum getrocknet. Man erhält 38 g Verbindung der Formel 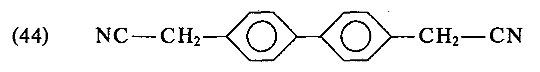 vom Schmelzpunkt 168°C, m/e = 232 (M^{⊕}).

### Beispiel 45

In einer Mischung aus 1,3 I Eisessig und 1,3 I Wasser werden 373 g Verbindung der Formel (33), als feuchter Preßkuchen (700 g), sowie 730 g Urotropin 2 Stunden unter Rückfluß zum Sieden erhitzt und abgekühlt. Der kristalline Niederschlag wird abgesaugt, mit Wasser gewaschen und bei 50°C im Vakuum getrocknet. Man erhält 252 g Verbindung der Formel 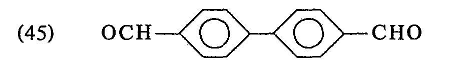 als farbloses Kristallpulver. Schmelzpunkt: 139°C (aus Di-n-butylether), m/e = 210 (M^{⊕}).

Die Mutterlauge kann vorteilhaft für Folgeansätze wiederverwendet werden, wobei man jeweils nur die Hälfte der ursprünglich verwendeten Urotropinmenge einzusetzen braucht. Nach DE-AS 1 794386, DE-OS 2 241 304 und DE-OS 3 001 876 können aus (45) wertvolle optische Aufheller der 4,4'-Distyryl-biphenyl-Reihe und nach DE-OS 2 901 845 wertvolle Papierfarbstoffe hergestellt werden.

In analoger Weise werden aus Verbindungen (34) und (35) die entsprechenden Dialdehyde der Formel 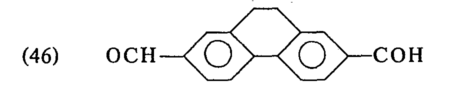 (m/e = 238, M^{⊕}) und 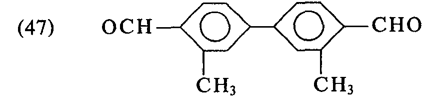 (m/e = 240, M )
hergestellt.

### Beispiel 48

300 g Verbindung der Formel (37) und 250 g Urotropin werden in 1,5 kg Eisessig und 70 ml Wasser 3 Std. unter Rückfluß zum Sieden erhitzt und abgekühlt. Der erhaltene Niederschlag wird abgesaugt, mit Methanol gewaschen und bei 50°C im Vakuum getrocknet. Man erhält 153 g Verbindung der Formel 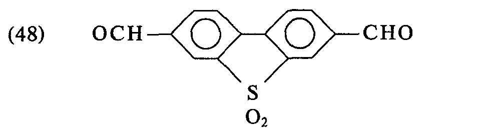 als farbloses Kristallpulver.

Schmelzpunkt: 302°C ; m/e = 272 (M ) (aus Dimethylformamid).

### Beispiel 49

In analoger Weise, jedoch ohne Schwefelsäurebehandlung wird Verbindung (37 a) in den Dialdehyd der Formel 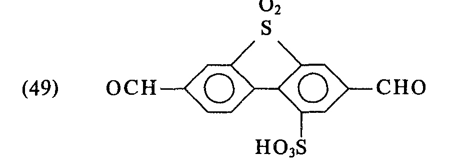 übergeführt und nach dem Ausfällen mit Isopropanol isoliert.

### Beispiel 50

6,6 g Verbindung der Formel (36) und 5,6 Urotropin werden in 90 ml Eisessig 3 Std. unter Rückfluß zum Sieden erhitzt, auf 30°C abgekühlt, mit 6,2 g p-Nitrophenylhydrazin versetzt, 30 Min. bei 30-40°C verrührt und auf 20°C abgekühlt. Der kristalline Niederschlag wird abgesaugt, mit Methanol gewaschen und bei 40°C im Vakuum getrocknet. Man erhält 8,8 g Verbindung der Formel 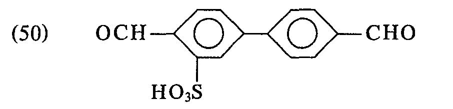 als Bis-p-nitrophenylhydrazon (orangefarbenes Kristallpulver, Schmelzpunkt 300°C).

### Beispiel 51

In 1 kg Oleum (20 %freies S0₃) trägt man bei Raumtemperatur unter Kühlung und Rühren 200 g Verbindung der Formel (1) ein und verrührt die Mischung 18 Stunden bei Raumtemperatur, wobei die Verbindung der Formel (30) entsteht. Diese wird ohne Zwischenisolierung zur Verbindung der Formel (37), hier mit 2 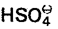 statt mit 2 Cl^{⊖}, verseift. Man geht dazu so vor, daß man unter starker Kühlung und unter Rühren 430 ml Wasser zutropft, die Mischung 8 Stunden auf 100°C erwärmt und abkühlt. Der kristalline Niederschlag wird abgesaugt, mit 600 ml 8 %ig. Kochsalzlösung gewaschen und bei 60°C im Vakuum getrocknet. Man erhält 128 g Verbindung der Formel (37) als 2 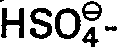 Salz.

### Beispiel 52

In 1 kg Oleum (20 %freies S0₃) trägt man bei Raumtemperatur unter Kühlung und Rühren 200 g Verbindung der Formel (1) ein und verrührt die Mischung 18 Stunden bei Raumtemperatur, wobei die Verbindung der Formel (30) entsteht. Diese wird ohne Zwischenisolierung zur Verbindung der Formel (32) (freie Sulfonsäure statt des Na-Salzes) sulfoniert und anschließend ohne Zwischenisolierung zur Verbindung (37a) ( 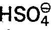 -Salz) verseift. Man geht dazu so vor, daß man 1 kg Oleum (65 %freies SO₃) zutropft, die Mischung 6 Stunden auf 80°C erwärmt, unter Kühlung und unter Rühren mit 1,6 Liter Wasser verdünnt, 8 Stunden auf 100°C erhitzt und abkühlt. Der kristalline Niederschlag wird abgesaugt, mit 600 ml 8 %ig. Kochsalzlösung gewaschen und bei 60°C im Vakuum getrocknet. Man erhält 136 g Verbindung der Formel (37 a) als 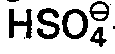 -Salz.

## Patentansprüche

1. Verbindungen der Formel 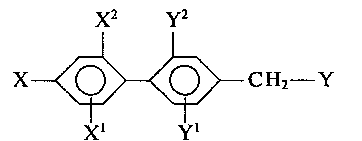 worin 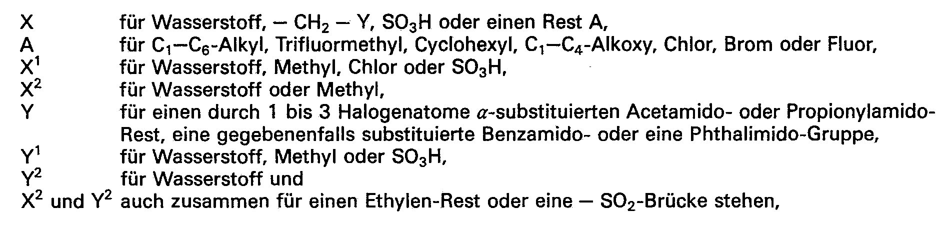
mit der Maßgabe, daß mindestens ein Rest Y für halogensubstituiertes Acetamido, Propionylamido oder für gegebenenfalls substituiertes Benzamido steht, wenn die Verbindungen frei von Sulfonsäuregruppen oder von - SO₂-Brücken sind.

2. Verbindungen des Anspruchs 1, dadurch gekennzeichnet, daß der Benzamidorest Y 1-3 Kernsubstituenten aus der Reihe C₁―C₄-Alkyl, Chlor, Brom, Methoxy, Ethoxy, Nitro und Cyan und/oder eine N― Cₗ-C₂-Alkylgruppe enthalten kann.

3. Verbindungen der Formel 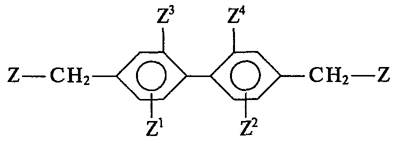 worin

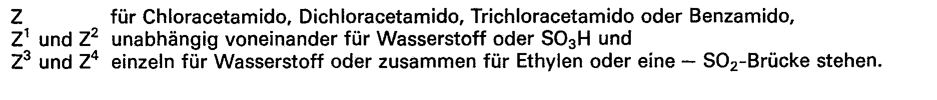

4. Verbindungen der Formel 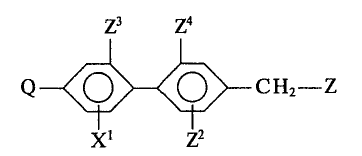 worin 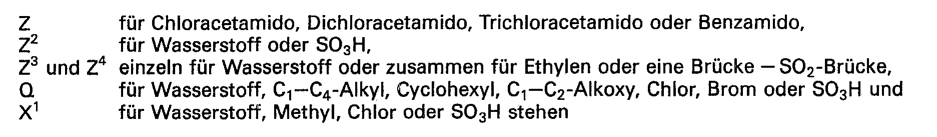
mit der Maßgabe, daß höchstens einer der Reste Q und X¹ SO₃H bedeutet.

5. Verfahren zur Herstellung von Verbindungen gemäß Anspruch 1, dadurch gekennzeichnet, daß man Verbindungen der allgemeinen Formel 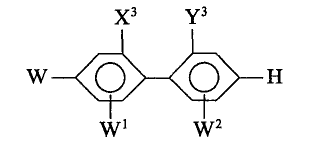 worin 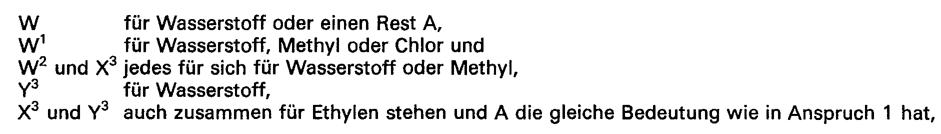
mit einem oder - im Falle W = H - gewünschtenfalls auch mit zwei Äquivalenten einer Verbindung der Formel 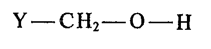 worin 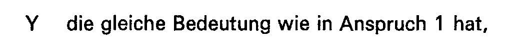
in Mischungen von Schwefelsäure und einer niederen Fettsäure im Temperaturbereich von-10°C bis + 50°C unter Abspaltung von Wasser kondensiert und gegebenenfalls anschließend mit einem sulfonierend wirkenden Mittel umsetzt.

## Claims

1. Compounds of the formula 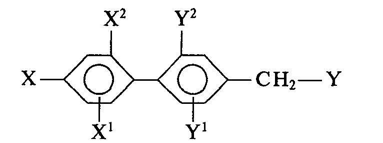 wherein 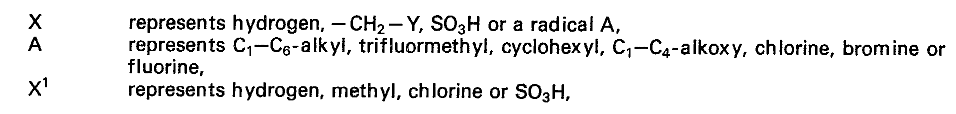 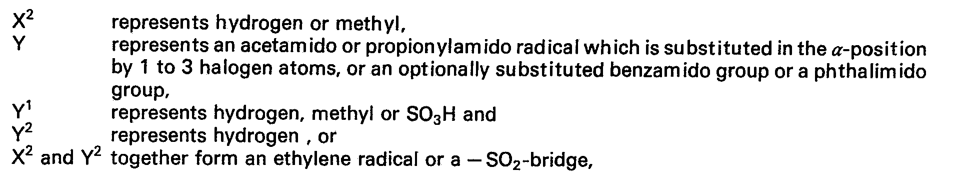
with the proviso that at least one radical Y represents halogen-substituted acetamido or propionylamido or optionally substituted benzamido if the compounds are free from sulphonic acid groups or from ―SO₂-bridges.

2. Compounds of Claim 1, characterised in that the benzamido radical Y can contain, on the nucleus, 1-3 substituents from the series comprising Cₗ-C₄-alkyl, chlorine, bromine, methoxy, ethoxy, nitro and cyano and/or one N―C₁―C₂-alkyl group.

3. Compounds of the formula 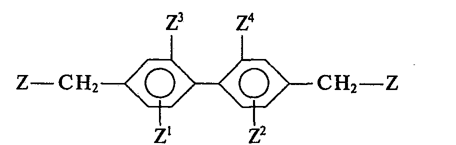 wherein

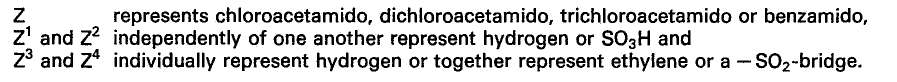

4. Compounds of the formula 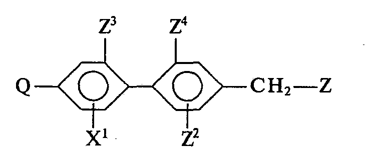 wherein 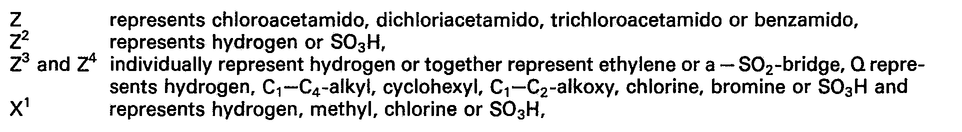
with the proviso that at most one of the radicals Q and X' denotes S0₃H.

5. Process for the preparation of compounds according to Claim 1, characterised in that compounds of the general formula 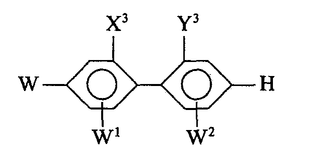 wherein 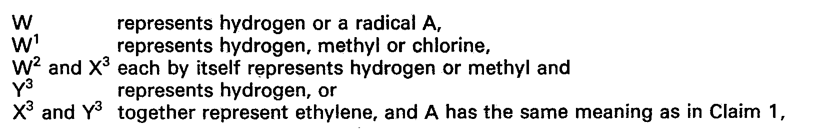
are subjected to a condensation reaction with one or - in the case where W = H - if desired also with two equivalents of a compound of the formula 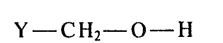 wherein 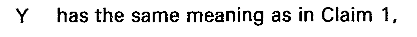
in mixtures of su Iphuric acid and a lower fatty acid in the temperature range of-10°C to + 50°C, water being split off, and, if appropriate, the product is then reacted with an agent having sulphonating action.

## Revendications

1. Composés de formule 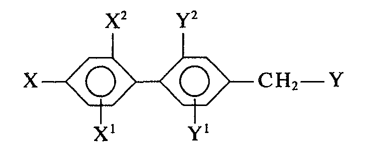 dans laquelle 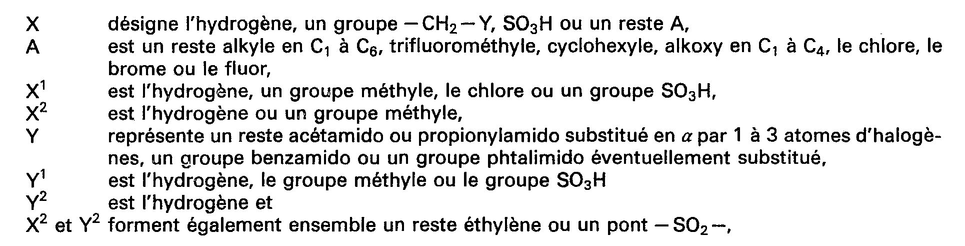
sous réserve qu'au moins un reste Y reprédente un groupe acétamido halogéné, propionylamido ou benzamido éventuellement substitué lorsque les composés sont dépourvus de groupes acide sulfoni- que ou de ponts ―SO₂―.

2. Composés suivant la revendication 1, caractérisés en ce que le reste benzamido Y peut comporter 1 à 3 substituants du noyau choisis dans la série alkyle en C₁ à C₄, chlore, brome, méthoxy, éthoxy, nitro et cyano et/ou un groupe N-(alkyle en Ci ou C_{z}).

3. Composés de formule 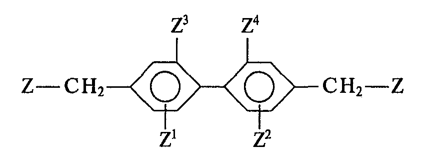 dans laquelle

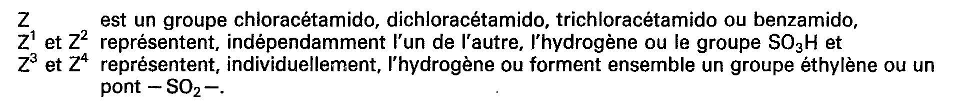

4. Composés de formule 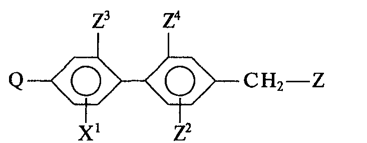 dans laquelle 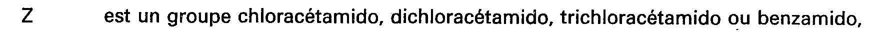 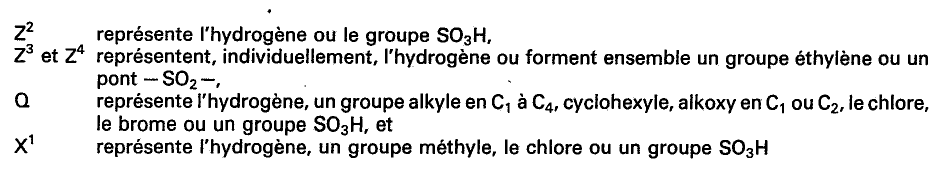
sous réserve qu'au maximum l'un des restes Q et X¹ représente un groupe SO₃H.

5. Procédé de production de composés suivant la revendication 1, caractérisé en ce qu'on condense des composés de formule générale 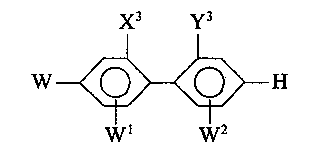 dans laquelle 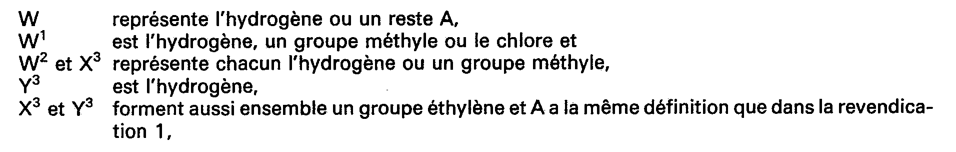
avec un ou - lorsque W représente l'hydrogène - en cas de besoin également deux équivalents d'un composé de formule 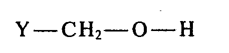 dans laquelle 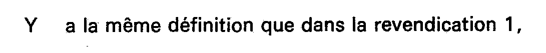
dans des mélanges d'acide sulfurique et d'un acide gras inférieur dans l'intervalle de température de -10°C à +50°C avec élimination d'eau, puis on les fait réagir éventuellement avec un agent de sulfonation.
